# EUROPÄISCHE PATENTSCHRIFT

(11) **EP 1 270 544 B1**
(45) Veröffentlichungstag und Bekanntmachung des Hinweises auf die Patenterteilung: **22.12.2004**
(21) Anmeldenummer: 02022029.9
(22) Anmeldetag: 26.01.1999
(51) Int. Cl.: C07C 209/78, C07C 263/10, C07C 211/50, C07C 265/14

(54) **Verfahren zur Herstellung von Methylendi (phenylamin) und Methylendi (phenylisocyanat)**
Process for the preparation of methylenedi(phenylamine) and methylenedi(phenylisocyanate)
Procédé de préparation de méthylènedi(phénylamine) et de méthylènedi(phénylisocyanate)

(30) Priorität: 07.02.1998 DE 19804915
(43) Veröffentlichungstag der Anmeldung: 02.01.2003
(62) Teilanmeldung aus: 99906202.9
(73) Patentinhaber: BASF AKTIENGESELLSCHAFT, 67056 Ludwigshafen (DE)
(72) Erfinder: Ströfer, Eckhard, Dr., 68163 Mannheim (DE); Jacobs, Jan D., Baton Rouge, LA 70810 (US); Seyfert, Wilfried, Dr., 1970 Wezembeek-Oppern (BE); Schwarz, Hans Volkmar, Dr., Baton Rouge, LA 70817 (US); Schweers, Olaf, Dr., 67316 Carlsberg (DE); Scharr, Volker, 01968 Senftenberg (DE); Penzel, Ulrich, Dr., 01945 Tettau (DE)

(56) Entgegenhaltungen:
- EP-A- 0 451 442
- DE-A- 2 134 756
- GB-A- 1 378 423
- US-A- 3 471 543

## Beschreibung

Die Erfindung betrifft Verfahren zur Herstellung von Methylendi(phenylisocyanat) durch Phosgenierung von Methylendi(phenylamin), hergestellt durch Umsetzung von Anilin mit Formaldehyd in Gegenwart saurer Katalysatoren, wobei man in einem halbkontinuierlichen Verfahren Anilin und gegebenenfalls sauren Katalysator vorlegt, Formaldehyd und gegebenenfalls sauren Katalysator durch ein Mischorgan in einen Kreislauf, in dem Anilin, gegebenenfalls saurer Katalysator und gegebenenfalls bereits zugegebenes Formaldehyd kreisläufig bewegt werden, einspeist und nach Einspeisung von mindestens 50 % der gesamt einzuspeisenden Formaldehydmenge die Reaktionsmischung auf eine Temperatur von größer 75°C temperiert, dadurch gekennzeichnet, dass man die Phosgenierung bei Temperaturen von 70 bis 120°C und einem Druck von 0,8 bis 5 bar in einer oder mehreren Stufen durchführt.

Die Herstellung von Methylendi(phenylamin), im Folgenden auch als MDA bezeichnet, ist allgemein bekannt und erfolgt üblicherweise durch kontinuierliche oder diskontinuierliche Umsetzung von Anilin mit Formaldehyd in Gegenwart von sauren Katalysatoren. Bei dieser Umsetzung, deren Hauptprodukt das 4,4'-MDA ist, wird in geringem Ausmaß das unerwünschte Nebenprodukt N-Methyl-MDA gebildet. Dieses Nebenprodukt wirkt sich insbesondere bei der anschließenden Umsetzung des MDAs mit Phosgen zur Herstellung von Methylendi(phenylisocyanat), auch als MDI bezeichnet, negativ aus, da das N-Methyl-MDA die Vorläuferverbindung für chlorierte Nebenprodukte im MDI darstellt und möglichst geringe Gehalte an Chlor im MDI angestrebt werden.

Zur Verringerung von N-Methyl-MDA als Nebenprodukt bei der Herstellung von MDA sind verschiedene Verfahren bekannt.

So beschreibt US 5 286 760 für eine kontinuierliche MDA- Herstellung eine Teilneutralisierung des Reaktionsgemisches zwischen der Kondensationsstufe von zwei Molekülen Anilin und einem Molekül Formaldehyd und der anschließenden Umlagerung der intermediär gebildeten Aminobenzylamine, abgekürzt ABA, zum MDA.

EP-A 451 442 und DD-A 238 042 offenbaren für ein kontinuierliches Verfahren die Zugabe von Formaldehyd über mehrere Verfahrensstufen.

Auch für diskontinuierliche Verfahren sind Verfahren zur Verringerung des Nebenproduktes bekannt. DD-A 295 628 beschreibt die Zugabe des Formaldehydes in zwei Schritten während der Kondensationsstufe, wobei in der ersten Zugabe die Hauptmenge des Formaldehydes bei niederer Temperatur erfolgt und die zweite Zugabe des restlichen Formaldehydes bei gleicher oder höherer Temperatur erfolgt.

Auch GB 1 378 423 und DE-A 2 134 756 offenbaren Verfahren zur Herstellung von MDA. US 3 471 543 beschreibt die Herstellung von Isocyanaten durch Phosgenierung von Aminen.

Nachteilig bei diesen Verfahren ist die nicht ausreichende Absenkung des Gehaltes an N-Methyl-MDA im Produktgemisch, so daß weiterhin ein Bedarf zur Verbesserung existiert.

Verfahren zur Herstellung von MDI aus MDA durch Phosgenierung sind allgemein bekannt.

Der vorliegenden Erfindung lag deshalb die Aufgabe zugrunde, ein Verfahren zur Herstellung von Methylendi(phenylamin) durch Umsetzung von Anilin mit Formaldehyd in Gegenwart saurer Katalysatoren zu entwickeln, durch das der Gehalt an N-Methyl-MDA als unerwünschtes Nebenprodukt minimiert wird. Ein solches MDA sollte insbesondere in einem verbesserten Verfahren zur Herstellung von Methylendi(phenylisocyanat) (MDI) eingesetzt werden, womit ein MDI mit verbesserten Eigenschaften, insbesondere einem geringen Chlorgehalt und/oder einer hellen Farbe, insbesondere im Roh-MDI, das neben dem monomeren MDI auch polymeres MDI enthält, und/oder in dem polymeren MDI zugänglich gemacht werden sollte.

Diese Aufgabe konnte erfindungsgemäß durch ein Verfahren zur Herstellung von Methylendi(phenylisocyanat) durch Phosgenierung von Methylendi(phenylamin), hergestellt durch Umsetzung von Anilin mit Formaldehyd in Gegenwart saurer Katalysatoren, wobei man in einem halbkontinuierlichen Verfahren Anilin und gegebenenfalls sauren Katalysator vorlegt, Formaldehyd und gegebenenfalls sauren Katalysator durch ein Mischorgan in einen Kreislauf, in dem Anilin, gegebenenfalls saurer Katalysator und gegebenenfalls bereits zugegebenes Formaldehyd kreisläufig bewegt werden, einspeist und nach Einspeisung von mindestens 50 % der gesamt einzuspeisenden Formaldehydmenge die Reaktionsmischung auf eine Temperatur von größer 75°C temperiert, dadurch gekennzeichnet, dass man die Phosgenierung bei Temperaturen von 70 bis 120°C und einem Druck von 0,8 bis 5 bar in einer oder mehreren Stufen durchführt, gelöst werden.

Diese erfindungsgemäße Fahrweise erlaubt es, einen höheren Anteil an höheren MDA-Oligomeren zu erhalten als es mit einer kontinuierlichen Fahrweise bei hohen molaren Verhältnissen von Anilin zu Formaldehyd ohne Rückführung des MDA möglich ist. Durch das erfindungsgemäße Verfahren ist eine Minimierung des Gehaltes an unerwünschten Nebenprodukten möglich.

Die erfindungsgemäße Umsetzung von Anilin mit Formaldehyd, bevorzugt in Gegenwart von sauren Katalysatoren, erfolgt erfindungsgemäß halbkontinuierlich, d.h. eine Reaktionskomponente, das Anilin und bevorzugt der saure Katalysator, wird vorgelegt und die zweite Reaktionskomponente, das Formaldehyd und gegebenenfalls saurer Katalysator, wird zu der ersten Reaktionskomponente zugegeben. Bevorzugt erfolgt das erfindungsgemäße Verfahren derart, daß man Anilin und sauren Katalysator vorlegt und Formaldehyd dieser ersten Reaktionskomponente zugibt. Die Umsetzung wird üblicherweise bei Temperaturen von 20 bis 150°C durchgeführt. Bevorzugt wird das erfindungsgemäße Verfahren derart durchgeführt, daß man die Zugabe des Formaldehydes zu dem Reaktionsgemisch im Kreislauf, d.h. dem Anilin, dem sauren Katalysator und gegebenenfalls bereits zugegebenem Formaldehyd und Reaktionsprodukten, bis zu einer Menge von mindestens 50 % der gesamt einzuspeisenden Formaldehydmenge, bevorzugt bis zur vollständigen Zugabe des gesamten Formaldehydes, bei einer Temperatur des Reaktionsgemisches im Kreislauf von 20 bis 75°C, bevorzugt 20 bis 60°C, besonders bevorzugt 30 bis 40°C, durchführt.

Die Temperatur hat einen Einfluß auf die Isomerenverteilung des Methylendi(phenylamins) im Produkt. Falls bevorzugt die 2,2'und/oder 2,4'-Methylendi(phenylamine) hergestellt werden sollen, kann eine hohe Temperatur vorteilhaft sein. Die Temperierung des Reaktionsgemisches kann mit allgemein üblichen Einrichtungen, z.B. mit Wärmetauschern im Umpumpkreis oder einem zweiten Umpumpkreis und/oder über die Wandung des Reaktors durchgeführt werden.

Das Reaktionsgemisch wird nach Einspeisung von mindestens 50 % der gesamt einzuspeisenden Formaldehydmenge in das Reaktionsgemisch, bevorzugt gegen Ende der Einspeisung der Formaldehydlösung, besonders bevorzugt nach vollständiger Zugabe des gesamten Formaldehydes zu dem Reaktionsgemisch, bevorzugt für eine Dauer von mindestens 0,2 Stunden, besonders bevorzugt 0,2 bis 48 Stunden, insbesondere 0,2 bis 6 Stunden auf eine Temperatur von größer 75°C, bevorzugt größer 90°C, besonders bevorzugt 105 bis 150°C, insbesondere 110 bis 135°C, temperiert. Besonders bevorzugt kann man nach vollständiger Zugabe des Formaldehydes zu der Reaktionsmischung das Reaktionsgemisch für eine Dauer von 0,1 bis 120 min auf eine Temperatur von 65 bis 100°C temperieren und anschließend, wie bereits beschrieben, auf eine Temperatur von größer 75°C erhitzen. Diese Temperierung bietet den Vorteil, daß die Handhabbarkeit des Reaktionsgemisches vereinfacht wird, da das Reaktionsgemisch bei der höheren Temperatur eine geringere Viskosität aufweist. Zudem werden bei dieser Temperierung in dem Reaktionsgemisch unerwünschte Nebenprodukte in einer Alterungsphase abgebaut oder umgelagert. Die Alterung des Reaktionsgemisches unter diesen bevorzugten Bedingungen kann in der Apparatur, in der die Reaktion des Formaldehydes mit dem Anilin durchgeführt wurde, ablaufen, als auch diskontinuierlich oder kontinuierlich in einer anderen Apparatur, in das die Reaktionsmischung nach der vollständigen Zugabe des Formaldehydes überführt werden kann. Beispielsweise kann das Reaktionsgemisch in der Apparatur, in der die Einspeisung der Formaldehydlösung erfolgt oder erfolgte, gealtert werden. Es ist auch möglich, das Reaktionsgemisch aus der Apparatur in mindestens einen weiteren Reaktor, beispielsweise einen Rohrreaktor und/oder Rührkessel, zu leiten, und in diesem Reaktor(en) die Alterung bei einer Temperatur von größer 75°C vorzunehmen. Bevorzugt wird das Reaktionsgemisch nach der vollständigen Zugabe des Formaldehydes in eine andere Apparatur überführt, in der dann die Alterung vervollständigt wird. Besonders bevorzugt wird das Reaktionsgemisch nach der vollständigen Zugabe des Formaldehydes, die bevorzugt bei einer Temperatur von 20 bis 60°C, besonders bevorzugt 30 bis 40°C, erfolgte, in einen üblichen Vorratsbehälter überführt, wie beschrieben bevorzugt auf eine Temperatur von 65 bis 100°C temperiert und anschließend in üblichen Reaktoren, bevorzugt einem Rohrreaktor, wie beschrieben bevorzugt auf eine Temperatur von 105 bis 150°C, besonders bevorzugt 110 bis 135°C erwärmt.

Das Reaktionsgemisch kann somit beispielsweise in Rohrreaktoren, Rührkesseln, Rührkesselkaskaden, Kombinationen aus Rührkesseln und Rohrreaktoren, geleitet werden, in denen gegebenenfalls die Umsetzung zum MDA vervollständigt werden kann.

Das Reaktionsgemisch enthaltend MDA sowie üblicherweise polymeres MDA kann nach der Umsetzung durch allgemein bekannte Verfahren aufgearbeitet werden, beispielsweise durch Neutralisation, Phasentrennung, Destillation und/oder chromatographische Trennmethoden, bevorzugt durch Neutralisation, bevorzugt bei einer Temperatur von 60 bis 110°C, und Entfernung von Wasser, Anilin und gegebenenfalls weiteren unerwünschten Begleitstoffen durch Destillation dieser Substanzen.

Bevorzugt neutralisiert man das Reaktionsgemisch, bevorzugt mit wäßriger Natronlauge, beispielsweise 50 %iger wäßriger Natronlauge, bevorzugt bei einer Temperatur von 60 bis 110°C, und trennt anschließend die wäßrige Phase durch Phasentrennung ab. Zur Entfernung von anorganischen Verunreinigungen kann man die organische Phase bei einer Temperatur von üblicherweise 60 bis 110°C mit Wasser waschen, die wäßrige Phase abtrennen und anschließend durch Destillation, bevorzugt bei einem Druck von 1050 bis 5 mbar und einer bevorzugten Temperatur von 180 bis 240°C, nicht umgesetztes Anilin aus der organischen Phase, d.h. dem MDA, entfernen.

Die Ausgangskomponenten Formaldehyd, Anilin und sauerer Katalysator können in üblichen Reinheitsgraden verwendet werden, wobei das Formaldehyd im Gleichgewicht mit höhermolekularen Anlagerungsprodukten wie zum Beispiel Poly(oxymethylen)glykolen stehen kann. Das Formaldehyd kann in üblichen, beispielsweise wässrigen Lösungen mit einem Gehalt von 10 bis 60 Gew.-% Formaldehyd, bezogen auf das Gewicht der Lösung, eingesetzt werden. Der Formaldehyd kann auch gasförmig zugeführt werden. In diesem Falle erfolgt die Zufuhr als Reingas oder im Gemisch mit Inertgas. Wasser kann je nach Bedarf getrennt zugegeben werden.

Die kreisläufige Bewegung des Reaktionsgemisches in einer geeigneten Apparatur kann durch allgemein übliche Einrichtungen, beispielsweise Pumpen, erfolgen. Die Geschwindigkeit, mit der das Reaktionsgemisch in dem Kreislauf bewegt wird, beträgt bevorzugt 1 bis 6 m/sec. Die Einspeisung der Formaldehydlösung kann über eine Reaktionsmischpumpe wie z.B. in DE-A 4220239 beschrieben oder über ein in den Pumpenkreislauf eingebautes Düsensystem, z.B. eine Ringspaltdüse erfolgen. Im Falle der Reaktionsmischpumpe dient die Einrichtung nicht nur zur Einspeisung des Formaldehydes und einer bevorzugt vollständigen Durchmischung, sondern auch zur Bewegung der Reaktionsmischung in der Apparatur. Wird eine Düse eingesetzt, so kann die Bewegung des Reaktionsgemisches in der Apparatur durch übliche, in der Chemie bekannte Pumpen erfolgen. Die in der Mischzone des Mischorgans, d.h. beispielsweise der Düse oder der Reaktionsmischpumpe, bei der Einspeisung des Formaldehydes in die Reaktionsmischung lokal dissipierte Mischenergie beträgt bevorzugt 100 bis 100000 W/1. Der in dem Kreislauf umgepumpte Mengenstrom steht zu dem in den Kreislauf eingespeisten Mengenstrom an Formaldehydlösung in einem Mengenverhältnis von bevorzugt mindestens 20:1.

Als saurer Katalysator können allgemein für diese Umsetzung bekannte Katalysatoren verwendet werden, beispielsweise Säuren mit einem pKs <1,5, z.B. Mineralsäuren wie Phosphorsäure, Schwefelsäure und/oder Salzsäure (HCl), bevorzugt wird HCl eingesetzt. Anilin und der saure Katalysator, bevorzugt HCl, werden bevorzugt bei einer Temperatur von 30 bis 60°C, bevorzugt 35 bis 45°C, gemischt,

Das molare Verhältnis von Anilin zu saurem Katalysator in dem Reaktionsgemisch beträgt üblicherweise 1:0,6 bis 1:0,01, bevorzugt 1:0,3 bis 1:0,05. Dieses molare Verhältnis gilt insbesondere für die besonders bevorzugte Ausführungsform, in der Anilin und saurer Katalysator vorgelegt werden und anschließend Formaldehyd und kein weiterer saurer Katalysator zugegeben wird.

Das molare Verhältnis von Anilin zu insgesamt zuzusetzendem Formaldehyd beträgt üblicherweise 1,7:1 bis 7,2:1, bevorzugt 1,9:1 bis 5,1:1, besonders bevorzugt 1,9:1 bis 3,6:1. Das Formaldehyd wird bevorzugt durch eine Düse oder eine Reaktionsmischpumpe in den Kreislauf einspeist. Um unerwünschte Parallelreaktionen zu Nebenprodukten zu vermeiden, erfolgt die Zugabe des Formaldehydes bevorzugt derart, daß eine möglichst rasche und vollständige Durchmischung mit dem Reaktionsgemisch, das sich in der Apparatur befindet, stattfindet. Dies kann beispielsweise durch die Erzeugung einer turbulenten Strömung in der Mischkammer erreicht werden.
In dem erfindungsgemäßen Verfahren werden bevorzugt in einer Apparatur Anilin und bevorzugt HCl als saurer Katalysator vorgelegt, gemischt, in einem Kreislauf bewegt, beispielsweise durch eine angeschlossene, übliche Pumpe, und diesem Reaktionsgemisch, bevorzugt durch eine Reaktionsmischpumpe oder Düse, Formaldehyd zugegeben. Die Zugabe des Formaldehydes kann derart erfolgen, daß konstante Volumina pro Zeiteinheit in das Reaktionsgemisch eingespeist werden, bis ein geeignetes molares Verhältnis von Anilin zu Formaldehyd in dem Reaktionsgemisch vorliegt. Bevorzugt erfolgt die Zugabe derart, daß pro Minute 0,05 bis 2 % des ursprünglichen Volumens des Anilins in der Apparatur als Volumen Formaldehydlösung in das Reaktionsgemisch geleitet werden. Anstatt der Einleitung des konstanten Volumens des Formaldehydes pro Zeiteinheit kann das Formaldehyd derart dem Reaktionsgemisch zudosiert werden, daß das Volumen des Formaldehydes, das pro Zeiteinheit zugegeben wird, gemäß einer mathematischen Funktion mit dem Fortschritt der Zugabe sinkt. Bevorzugt ist eine konstante, linear fallende oder stufig fallende Zugaberate. Des Weiteren kann man das Formaldehyd gepulst in der Reaktionsgemisch einführen, wobei eine regelmäßige oder unregelmäßige Pulsfrequenz und Zugabemenge gewählt werden kann. Die insgesamt einzuleitende Formaldehydmenge sollte bevorzugt den eingangs beschriebenen molaren Verhältnissen in Bezug auf die Anilinmenge entsprechen. Bei dieser diskontinuierlichen Verfahrensweise wird das Reaktionsgemisch nach gewünschtem Umsatz aus der Apparatur entleert und gegebenenfalls weiter aufgearbeitet.

Die erfindungsgemäße Umsetzung kann beispielsweise in einer Apparatur durchgeführt werden, die
1: Zuleitungen für Anilin und sauren Katalysator,
2: Zuleitung für Formaldehyd,
3: mindestens ein Mischorgan, beispielsweise Reaktionsmischpumpe oder Düse, durch die das Formaldehyd in die Apparatur eingespeist wird,
4: mindestens einen Reaktor mit
5: fakultativ Einrichtungen zur Durchmischung des Reaktionsgemisches,
6: ein Rohrsystem, das ausgehend vom Reaktor einen Kreislauf des Reaktionsgemisches ermöglicht,
7: eine Einrichtung zur Temperierung des Reaktionsgemisches und
8: gegebenenfalls eine Pumpe, die das Reaktionsgemisch in (6) in einem Kreislauf bewegt,
9: mindestens einen Anschluß zur Entnahme des Reaktionsgemisches aufweist.

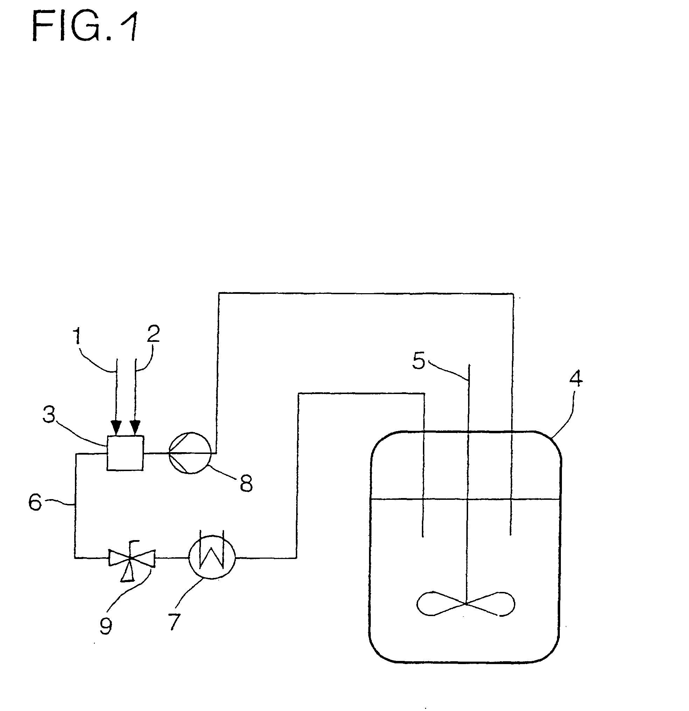

Eine solche Apparatur ist beispielhaft in Figur 1 dargestellt, wobei anzumerken ist, daß die Zugabe von Anilin und saurem Katalysator sowohl gemeinsam, wie in Figur 1 dargestellt, als auch getrennt, an weitgehend beliebiger Stelle der Apparatur, beispielsweise durch Zugabe in den Reaktor (4) oder durch Anschlüsse an der Reaktionsmischpumpe oder Düse (3), erfolgen kann. Auch die Einrichtungen 7, 8 und insbesondere 9 können weitgehend beliebig, beispielsweise im Falle des Anschlusses 9 auch am Reaktor 4 angeordnet sein.

Das Volumen des Reaktors (4) kann je nach gewünschtem Umsatz variabel gewählt werden. Auch der Durchmesser, der variabel gewählt werden kann, und die Länge des Rohrsystems (6) können je nach Ansatzgröße weitgehend frei gewählt werden. Für die Komponenten (1) bis (9) können, wie für die Komponenten (3) und (7) bereits dargestellt, übliche Einrichtungen verwendet werden. Eine zur Durchführung des erfindungsgemäßen Verfahrens geeignete Apparatur kann aus für diesen Zweck üblichen Materialien, beispielsweise Stahl/Email oder Edelstahllegierungen bestehen.

Das Verfahrensprodukt, das üblicherweise auch als Roh-MDA bezeichnet wird, d.h. das Gemisch enthaltend Methylendi(phenylamin), beispielsweise 2,2'-, 2,4'-, und/oder 4,4'-MDA als monomers MDA, und üblicherweise polymeres MDA, auch als Polymethylendi-(phenylamin) bezeichnet, enthält bevorzugt weniger als 0,09 Gew.-% N-Methyl MDA und wird bevorzugt zur bekannten Synthese von Methylen(diphenylisocyanat), bekannt als MDI oder Diphenylmethandiisocyanat, beispielsweise 2,2'-, 2,4'- und/oder 4,4'-MDI, und polymerem MDI, beispielsweise durch übliche Phosgenierung von Polyaminen eingesetzt.

Die Phosgenierung kann bevorzugt in üblichen, besonders bevorzugt inerten Lösungsmitteln, z.B. chlorierten, aromatischen Kohlenwasserstoffen, beispielsweise Monochlorbenzol, Dichlorbenzole wie z.B. o-Dichlorbenzol, p-Dichlorbenzol, Trichlorbenzole, die entsprechende Toluole und Xylole, Chlorethylbenzol, Monochlordiphenyl, alpha- bzw. beta-Naphthylchlorid und Phthalsäuredialkylester, wie iso-Diethylphthalat, bevorzugt Toluol, Mono- und/oder Dichlorbenzol, in üblichen Reaktoren, beispielsweise Rührkesseln, Rührkesselkaskaden, Kolonnen und/oder Rohrreaktoren bei Temperaturen von 70 bis 120°C, besonders bevorzugt 70 bis 100°C und einem Druck von 0,8 bis 5 bar, besonders bevorzugt 0,8 bis 1,5 bar in einer oder mehreren Stufen durchgeführt werden.

Beispielsweise kann die Phosgenierung durchgeführt werden durch eine zweistufige Umsetzung in Gegenwart mindestens eines inerten organischen Lösungsmittels, wobei die erste Stufe der Phosgenierung in einem statischen Mischer und die zweite Stufe der Phosgenierung in einem Verweilzeitapparat durchgeführt werden und im Verweilzeitapparat die Massenverhältnisse von Phosgen zu Chlorwasserstoff gleichzeitig in der Flüssigphase 10-30:1 und in der Gasphase 1-10:1 betragen.

Als statische Mischer für die erste Stufe der Phosgenierung kommen die bekannten und oben aufgeführten Vorrichtungen, insbesondere Düsen, zur Anwendung. Die Temperatur bei der ersten Stufe des Phosgenierung beträgt üblicherweise 50 bis 120°C, bevorzugt 60 bis 120°C, besonders bevorzugt 90 bis 120°C.

Das Gemisch der ersten Stufe der Phosgenierung wird bevorzugt einem Verweilzeitapparat zugeführt, wobei die Massenverhältnisse von Phosgen zu Chlorwasserstoff im Verteilzeitapparat der zweiten Stufe der Phosgenierung gleichzeitig in der Flüssigphase 10-30:1 und in der Gasphase 1-10:1 betragen.

Als Verweilzeitapparat für das erfindungsgemäße Verfahren kommen die bekannten Apparate zur Anwendung, vorzugsweise Rührmaschinen, insbesondere Rührkesselkaskaden mit 2 bis 6 Rührkesseln, oder Kolonnen, insbesondere solche mit < 10 theoretischen Böden.

Bei der Verwendung von Rührmaschinen als Verweilzeitapparate werden, wie oben ausgeführt, insbesondere Rührkesselkaskaden mit mindestens 2, vorzugsweise 2 bis 6, besonders bevorzugt 2 bis 5 Rührkesseln, eingesetzt. Prinzipiell ist auch eine Kaskade mit mehr als 6 Rührkesseln einsetzbar, eine Vergrößerung der Zahl der Rührkessel über 6 steigert jedoch nur noch den apparativen Aufwand, ohne daß eine meßbare Verbesserung des Endproduktes eintritt. Das Gemisch der ersten Stufe der Phosgenierung tritt üblicherweise mit einer Temperatur von 70-120°C, bevorzugt 85-105°C in die erste Rührmaschine ein. Die Temperaturen in den Rührmaschinen betragen bevorzugt, gemeinsam oder einzeln unterschiedlich, 75-120°C, besonders bevorzugt 80-110°C. Die Drücke in den Rührmaschinen betragen üblicherweise einzeln unterschiedlich oder gemeinsam 1,0-3,0 at (Ü), bevorzugt 1,2-2,5 at (Ü).

Besonders bevorzugt ist die Verwendung einer Kolonne als Verweilzeitapparat. Hierbei ist es besonders vorteilhaft, die Kolonne im Gegenstrom zu betreiben. Das Produktgemisch der ersten Stufe der Phosgenierung wird bevorzugt so in die Kolonne eingespeist, daß das monomere MDI/gegebenenfalls polymere MDI/Lösungsmittel/ Phosgen-Gemisch die Kolonne über den Sumpf verläßt und ein Phosgen/Chlorwasserstoffgemisch über Kopf der Kolonne abgezogen wird und der Chlorwasserstoff/Phosgen-Trennung zugeführt wird. Die Eintrittstemperatur des Gemisches der ersten Stufe der Phosgenierung in die Kolonne kann bevorzugt 80-120°C, besonders bevorzugt 82-117°C, betragen. Die Sumpftemperatur der Kolonne beträgt dabei vorzugsweise 80-120°C, besonders bevorzugt 90-110°C. Der Kopfdruck der Kolonne beträgt vorzugsweise 1,0-4,7 at (Ü), besonders bevorzugt 2,0-3,7 at (Ü). Das Chlorwasserstoff/Phosgenverhältnis in der Kolonne wird bevorzugt durch den Phosgenüberschuß in der ersten Stufe der Phosgenierung, der Eintrittstemperatur des Reaktionsproduktes in die Kolonne, dem Kolonnendruck und der Sumpftemperatur der Kolonne eingestellt und kontrolliert. Die Phosgenmenge kann insgesamt der ersten Stufe der Phosgenierung zugeführt werden oder nur teilweise, wobei . in diesem Fall eine weitere Menge in den Verweilzeitapparat der zweiten Stufe der Phosgenierung eingespeist wird. Die verwendete Kolonne hat vorzugsweise < 10 theoretischen Böden. Vorteilhaft ist die vorzugsweise Verwendung einer Ventilbodenkolonne. Es sind auch andere Kolonneneinbauten geeignet, die die notwendige Verweilzeit für die Carbamylchloridspaltung sowie eine schnelle und effektive Chlorwasserstoffentfernung gewährleisten, wie z.B. Glockenbodenkolonnen, Destillationsböden mit erhöhten Flüssigkeitswehren. Die in DE-A 3 744 001 vorgeschlagene Lochbodenkolonne kann die Aufgabe der schonenden Carbamylchloridspaltung bei schneller und effektiver Chlorwasserstoffentfernung technisch nur sehr unzureichend erfüllen.

Die nach dem erfindungsgemäßen Verfahren hergestellten Mischungen (Roh-MDI), enthaltend Diphenylmethandiisocyanate (monomeres MDI) und Polyphenylen-polymethylen-polyisocyanate (polymeres MDI) besitzen üblicherweise einen Diphenylmethandiisocyanat-Isomerengehalt von 30 bis 90 Gew.-%, vorzugsweise von 30 bis 70 Gew.-%, einen NCO-Gehalt von 29 bis 33 Gew.-%, vorzugsweise 30 bis 32 Gew.-%, bezogen auf das Roh-MDI-Gewicht, und eine Viskosität, bestimmt gemäß DIN 51550 bei 25°C, von bevorzugt maximal 2500 mPa.s, vorzugsweise von 40 bis 2000 mPa.s.

Die Menge an Lösungsmittel bei der Phosgenierung wird zweckmäßig so bemessen, daß die Reaktionsmischung einen Isocyanatgehalt von 2 bis 40 Gew.-%, vorzugsweise zwischen 5 und 20 Gew.-%, bezogen auf das Gesamtgewicht der Reaktionsmischung, aufweist.

Das Phosgen kann als solches oder in Verdünnung mit unter den Reaktionsbedingungen inerten Gasen, wie Stickstoff, Kohlenmonoxid u.a. eingesetzt werden. Das Molverhältnis von Roh-MDA zu Phosgen wird zweckmäßigerweise so bemessen, daß pro Mol NH₂-Gruppe 1 bis 10 Mol, vorzugsweise 1,3 bis 4 Mol, Phosgen in der Reaktionsmischung vorliegen. Bei einem zweistufigen Verfahren kann die Phosgenmenge vollständig der ersten Stufe der Phosgenierung zugeführt werden oder teilweise auch dem Verweilzeitapparat der zweiten Stufe der Phosgenierung zugesetzt werden.

Das durch die Phosgenierung hergestellte Roh-MDI kann durch übliche Verfahren, beispielsweise Destillation, gereinigt werden. Bevorzugt kann in einem ersten Reinigungsvorgang Phosgen und gegebenenfalls Lösungsmittel, bevorzugt weitgehend, besonders bevorzugt vollständig aus dem Reaktionsgemisch der Phosgenierung, d.h. dem Roh-MDI entfernt werden. Dieser Reinigungsschritt kann bevorzugt durch einen Stripprozeß durchgeführt werden. Bei einem solchen Stripprozeß kann das Roh-MDI in einen oder mehrere Apparate mit großer innerer Oberfläche geleitet und auf dessen Oberfläche verteilt werden, so daß leichtflüchtige Komponenten entweichen können. Es kann sich bei der Apparatur beispielsweise und bevorzugt um einen Fallfilm- oder Dünnschichtverdampfer oder eine gepackte Kolonne geeigneter Auslegung handeln. Inertgase können als Stripmedium eingespeist und/oder Vakuum über die Apparatur angelegt werden. Die Temperaturen während dieses Stripprozesses betragen bevorzugt unter 210°C, besonders bevorzugt 50 bis 190°C. Bevorzugt kann gewünschtes monomeres MDI, beispielsweise 2,2'-, 2,4'- und/oder 4,4'-MDI und/oder Gemische enthaltend mindestens zwei diese Isomere, durch ein geeignetes Verfahren, bevorzugt durch Destillation, beispielsweise bei Drücken von 2 bis 50 mbar, bevorzugt 2 bis 20 mbar, und Temperaturen von 150 bis 250°C, bevorzugt 180 bis 230°C, und/oder bevorzugt durch Kristallisation, beispielsweise fraktionierte Kristallisation, abgetrennt werden.

Besonders bevorzugt führt man die Reinigung des Roh-MDIs derart durch, daß man bei einer Temperatur von <150°C, bevorzugt 50 bis 149°C, Phosgen, HCl und gegebenenfalls Lösungsmittel beispielsweise in einem bereits beschriebenen Stripprozeß, gegebenenfalls unter Vakuum oder Einspeisung von Inertgas, aus dem Roh-MDI entfernt, nach bevorzugt vollständiger Entfernung des Phosgens Lösungsmittel und gegebenenfalls chlorhaltige Verbindungen bei einer Temperatur von ≤209°C, bevorzugt 150 bis 209°C, besonders bevorzugt ≤190°C, insbesondere 150 bis 190°C, beispielsweise in einem bereits beschriebenen Stripprozeß, aus dem Isocyanat abtrennt, wobei die Reinigungsschritte mit den bereits beschriebenen Apparaturen durchgeführt werden können. Anschließend kann gewünschtes monomeres MDI, beispielsweise 2,2'-, 2,4'- und/oder 4,4'-MDI und/oder Gemische, enthaltend mindestens zwei diese Isomere, durch ein geeignetes Verfahren, bevorzugt durch Destillation, beispielsweise bei Drücken von 2 bis 50 mbar, bevorzugt 2 bis 20 mbar, und Temperaturen von 150 bis 250°C, bevorzugt 180 bis 230°C, besonders bevorzugt 210 bis 230°C, und/oder bevorzugt durch Kristallisation, beispielsweise fraktionierte Kristallisation, abgetrennt werden. Die Trennung des monomeren MDIs vom polymeren MDI erfolgt somit bevorzugt durch Destillation und/oder Kristallisation.

Danach wird das monomere MDI und/oder das polymere MDI üblicherweise mit einem Antioxidans auf Basis sterisch gehinderter Phenole und/oder mindestens einem Arylphosphit stabilisiert. Die Stabilisatoren werden zweckmäßigerweise in einer Menge bis max. 1 Gew.-%, vorzugsweise von 0,001 bis 0,2 Gew.-% eingesetzt.

Als geeignete Antioxidantien auf Basis sterisch gehinderter Phenole kommen beispielsweise in Betracht: Styrolisierte Phenole, d.h. Phenole, die in 2- oder 4-Stellung oder in 2- und 4-und/oder 6-Stellung eine 1-Phenyl-ethylgruppe gebunden enthalten, Bis-[2-hydroxy-5-methyl-3-tertbutylphenyl-]-methan, 2,2-Bis-[4-hydroxyphenyl]-propan, 4,4'-Dihydroxy-biphenyl, 3,3'-Dialkyl- bzw. 3,3', 5,5'-Tetraalkyl-4,4'-dihydroxy-biphenyl, Bis-[4-hydroxy-2-methyl-5-tert.-butylphenyl]-sulfid, Hydrochinon, 4-Methoxy-, 4-tert.-Butoxy- oder 4-Benzyloxyphenol, Gemische aus 4-Methoxy-2- bzw. -3-tert.-butylphenol, 2,5-Dihydroxy-1-tert.-butylbenzol, 2,5-Dihydroxy-1,4-di-tert.-butylbenzol, 4-Methoxy-2,6-di-tert.-butylphenol und vorzugsweise 2,6-Di-tertbutyl-p-kresol.

Als Arylphosphite bewährt haben sich Tri-(alkylphenyl)-phosphite mit 1 bis 10 C-Atomen im Alkylrest, wie z.B. Tri-(methylphenyl)-, Tri-(ethylphenyl)- Tri-(n-propylphenyl)-, Tri-(isopropylphenyl)-, Tri-(n-butylphenyl)-, Tri-(sek.-butylphenyl), Tri-(tert.-butylphenyl, Tri-(pentylphenyl)-, Tri-(hexylphenyl)-, Tri-(2-ethylhexylphenyl)-, Tri-(oktylphenyl)-,Tri-(2-ethyl-octylphenyl)-, Tri-(decylphenyl)-phosphit und vorzugsweise Tri-(nonylphenyl)-phosphit, und insbesondere Triphenylphosphit.

Diese Reinigungsprozesse bieten den Vorteil, daß chlorhaltige Verbindungen, die zu negativen Eigenschaften in dem gewünschten Isocyanat führen, aus dem Isocyanat entfernt werden und gleichzeitig die Ausbildung farbgebender Komponenten zurückgedrängt wird. Insbesondere das Roh-MDI und nach der Abtrennung der Monomeren, d.h. 2,2'-, 2,4'- und/oder 4,4'-MDI, aus dem Roh-MDI das polymere MDI im Destillationssumpf weisen erfindungsgemäß eine helle Farbe und einen niedrigen Chlorgehalt auf.

Das erfindungsgemäße Verfahren zur Herstellung von Methylendi(phenylisocyanat) kann somit derart durchgeführt werden, daß man in einem halbkontinuierlichen Verfahren Anilin und sauren Katalysator vorlegt, wobei das molare Verhältnis von Anilin zu saurem Katalysator 1:0,6 bis 1:0,01 beträgt, Formaldehyd durch eine Düse oder eine Reaktionsmischpumpe in einen Kreislauf, in dem Anilin, saurer Katalysator und gegebenenfalls bereits zugegebenes Formaldehyd kreisläufig bei einer Temperatur von 20 bis 75°C bewegt werden kann, einspeist, nach Einspeisung von mindestens 50 % der gesamt einzuspeisenden Formaldehydmenge die Reaktionsmischung für eine Dauer von mindestens 0,2 Stunden auf eine Temperatur von größer 75°C temperiert, wobei das molare Verhältnis von vorgelegtem Anilin zu insgesamt zuzusetzendem Formaldehyd 1,7:1 bis 7,2:1 beträgt, das so erhaltene Methylendi(phenylamin) neutralisiert, Wasser und Anilin abtrennt, das gereinigte Methylendi(phenylamin) bei einer Temperatur von 50 bis 150°C und einem Druck von 0,5 bis 10 bar, gegebenenfalls in Gegenwart von inerten Lösungsmitteln phosgeniert, Phosgen, HCl und gegebenenfalls Lösungsmittel beispielsweise in einem bereits beschriebenen Stripprozeß, gegebenenfalls unter Vakuum oder Einspeisung von Inertgas, bei einer Temperatur von <150°C aus dem Roh-MDI entfernt, anschließend bei einer Temperatur von ≤190°C Lösungsmittel und gegebenenfalls chlorhaltige Verbindungen beispielsweise in einem bereits beschriebenen Stripprozeß aus dem Isocyanat abtrennt und anschließend gewünschtes monomeres MDI, beispielsweise 2,2'-, 2,4'- und/oder 4,4'-MDI und/oder Gemische enthaltend mindestens zwei dieser Isomere, durch ein geeignetes Verfahren, bevorzugt durch Destillation, beispielsweise bei Drücken von 2 bis 50 mbar, bevorzugt 2 bis 20 mbar, und Temperaturen von 150 bis 250°C, bevorzugt 180 bis 230°C, und/oder bevorzugt durch Kristallisation, beispielsweise fraktionierte Kristallisation, abtrennt.

Das MDA und/oder das polymere MDA, beispielsweise das Roh-MDA, kann vor der Phosgenierung bei einer Temperatur von 100 bis 130°C gelagert werden.

Die mit dem erfindungsgemäßen Methylendi(phenylamin) hergestellten Polyisocyanate weisen insbesondere den Vorteil auf, daß sie einen geringen Gehalt an hydrolisierbarem Chlor besitzen. Zudem weist das erfindungsgemäß hergestellte Isocyanat eine gewünscht sehr helle Farbe auf. Diese Vorteile sind nicht nur in der erfindungsgemäßen Herstellung der Methylendi(phenylamine) mit dem geringen Gehalt an Nebenprodukten begründet, sondern auch darauf zurückzuführen, daß die Phosgenierung der Amine und die Aufarbeitung des Produktes bei geringen Drücken und damit geringen Temperaturen durchgeführt wird. Diese definierte Kombination vieler Prozeßparameter ausgehend vom Anilin bis zum fertigen Diisocyanat führt zu den erfindungsgemäß besonders vorteilhaften Produkten.

Bevorzugt weisen die erfindungsgemäß erhältlichen Isocyanate und Polyisocyanate, beispielsweise das Roh-MDI, das monomere MDI und das polymere MDI, besonders das Roh-MDI, insbesondere das polymere MDI, einen Gehalt an hydrolysierbarem Chlor von <0,1 %, besonders bevorzugt <0,045 %, und eine Iodfarbzahl in einer Verdünnung von 1:5 in Monochlorbenzol von <30, besonders bevorzugt <11, auf.

Das erfindungsgemäße Verfahren soll anhand der folgenden Beispiele näher dargestellt werden.

### Vergleichsbeispiel 1

Die Umsetzung wurde in einer Apparatur durchgeführt, die aus einer Rührkesselkaskade mit drei Reaktoren, die Volumina von 700, 800 und 800 ml auswiesen, und einem mit Füllkörpern gepackten Rohr bestand. Die Reaktionstemperaturen in den Reaktoren wurden mittels externer Kühlung bzw. Heizung auf 40 (erster Rührkessel), 70 (zweiter Rührkessel), 80 (dritter Rührkessel) und 120°C (Rohrreaktor) eingestellt. Das mit Füllkörpern gepackte Rohr wies ein Gesamtvolumen von 5000 ml und einen inneren Rohrdurchmesser von 30 mm auf. Die Drehzahl der Rührer in den Reaktoren der Rührkesselkaskade betrug jeweils 500 U/min. In den ersten Reaktor wurde Anilin mit 1264 g/h zugegeben, welches zuvor mit 422 g/h 30 %iger wässriger Salzsäure vermischt worden war. An dem ersten Reaktor befand sich ein äußerer Umpumpkreis mit einem statischen oder dynamischen Mischer, in den 341 g/h einer 50%ige Formaldehydlösung in Wasser mittels eine Pumpe eingegeben wurde. Das Produktgemisch aus dem Rohrreaktor wurde Natronlauge neutralisiert. Anschließend wurde eine Phasentrennung bei einer Temperatur von 70 bis 80°C vorgenommen. Die organische Phase wurde abgetrennt, mit dem 1,5 fachen Volumen an warmen Wasser gewaschen. Aus dieser gereinigten Phase wurde überschüssiges Anilin unter vermindertem Druck abdestilliert und in den ersten Reaktor zurückgeführt. 24 h nach dem Anfahren der Anlage war die Reaktionsmischung in einem stationären Zustand und es wurden Proben der organischen Phase genommen. Der Gehalt an N-Methyl MDA in dem erhaltenen Produkt betrug 0,26 Gew.-%. Diese Polyamin wurde in einem üblichen Verfahren zur Herstellung von Isocyanaten in einem zweistufigen Prozeß mit Phosgen umgesetzt. Der Gehalt an hydrolysierbare Chlor in diesem Polyisocyanat betrug 0,22 %.

### Beispiel 1

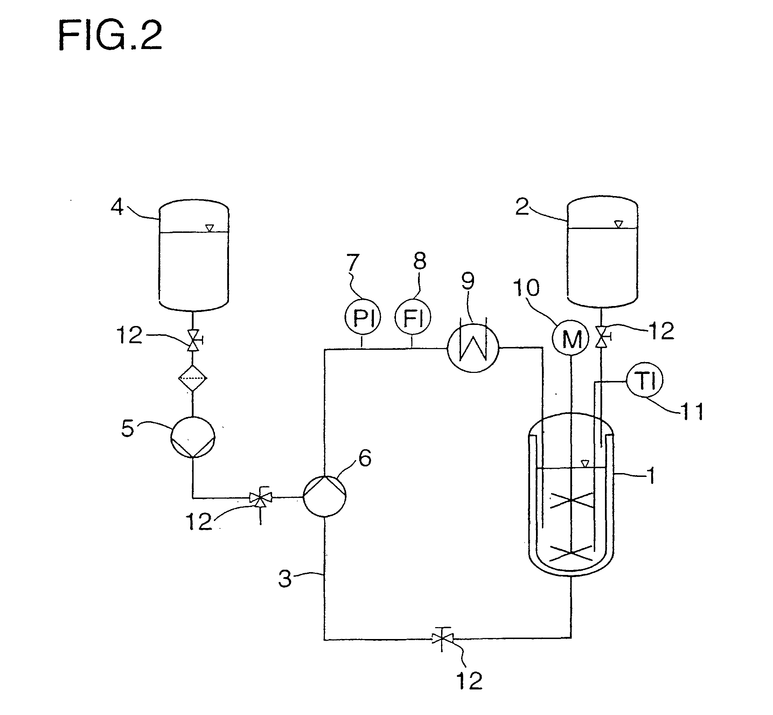

Es wurden in einer Apparatur gearbeitet wie in Figur 2 dargestellt. In dieser Figur 2 bedeuten:
1: Reaktor
2: Vorratsbehälter, Zulauf für Anilin und HCl
3: Umlauf, Kreislauf des Reaktionsgemisches
4: Vorratsbehälter, Zulauf für Formaldehydlösung
5: Zudosierpumpe
6: Mischorgan, Zuführung der Formaldehydlösung
7: Druckmessung
8: Flußmessung
9: Wärmetauscher
10: Rührer
11: Temperaturmessung
12: Hahn

Der Reaktor 1 hatte ein Volumen von 1000 ml. Die Drehzahl des Rührers betrug 500 U/min. Der äußere Umlauf 3, Umlaufrate des Reaktionsgemisches ca. 130 l/h, wird mit einer Pumpe betrieben. Es aus wurden aus dem Vorratsbehälter 735 g Anilin vorgelegt und im Reaktor 1 mit 243 g 30 %iger wässriger Salzsäure vermischt. Bei einer Temperatur von 40°C wurde nun innerhalb einer Stunde mit konstanter Dosierrate insgesamt 204 g einer 50 %igen Lösung von Formaldehyd in Wasser über das Mischorgan 6, einen dynamischen Mischer, in den Kreislauf zugegeben. Direkt nach der Zugabe der Formaldehydlösung wurde das Reaktionsgemisch aufgeheizt und anschließend 2,5 Stunden bei 120°C temperiert. Das Reaktionsgemisch wurde wie im Vergleichsbeispiel 1 beschrieben aufgearbeitet. Der Gehalt an N-Methyl MDA in dem erhaltenen Produkt betrug 0,07 Gew.-%. Diese Polyamin wurde in dem erfindungsgemäßen Verfahren zur Herstellung von Isocyanaten in einem zweistufigen Prozeß mit Phosgen umgesetzt. Der Gehalt an hydrolysierbarem Chlor in diesem Polyisocyanat betrug 0,06 %. 15

### Beispiel 2

Es wurden entsprechend Beispiel 1 verfahren, allerdings erfolgte die Zugabe der Formaldehydlösung in einer Stufenfunktion. In den ersten 30 min der Zugabe der Formaldehydlösung wurde mit einer Rate von 306 g/h zu dem Reaktionsgemisch zudosiert, in den zweiten 30 min mit einer Rate von 102 g/h. Das Reaktionsgemisch wurde wie im Beispiel 1 beschrieben aufgearbeitet. Der Gehalt an N-Methyl MDA in dem erhaltenen Produkt betrug 0,08 Gew.-%. Diese Polyamin wurde in einem Verfahren zur Herstellung von Isocyanaten in einem zweistufigen Prozeß mit Phosgen bei einer Temperatur von 80°C und einem Druck von 1 bar umgesetzt. Der Gehalt an hydrolysierbarem Chlor in diesem Polyisocyanat betrug 0,07 %. Die Iodfarbzahl des Isocyanates in einer Verdünnung von 1:5 mit Monochlorbenzol betrug 15.

Die Aufgabe, ein Verfahren zu entwickeln, mit dem die unerwünschte Bildung von N-Methyl MDA vermindert wird, konnte somit durch das erfindungsgemäße Verfahren gelöst werden. Nicht nur der Gehalt an dem unerwünschten N-Methyl MDA konnte deutlich um 73 bzw. 69 % verringert werden, auch der Gehalt an hydrolysierbarem Chlor in dem Polyisocyanat, das mit dem erfindungsgemäß hergestellten MDA produziert wurde, konnte drastisch um >70 % reduziert werden. Auch die Aufgabe, ein möglichst helles Isocyanat ausgehend von MDA herzustellen, konnte erreicht werden.

Sowohl das erfindungsgemäß hergestellte MDA als auch das mit diesem MDA produzierte Polyisocyanat wies somit wesentlich verbesserte Eigenschaften auf.

### Beispiel 3

MDA wurde in einer Apparatur hergestellt, wie sie in Figur 2 dargestellt ist und im Beispiel 1 beschrieben ist. Der Reaktor hatte ein Volumen von 45 m³. Der Vorratsbehälter 2 wurde mit einem Gemisch aus 17130 kg Anilin und 5378 kg 30 %iger wäßriger Salzsäure gefüllt, das anschließend in den Reaktor 1 überführt wurde. Die Drehzahl des Rührers betrug 70 U/min. Das Reaktionsgemisch wurde in dem Umlauf 3 bewegt. Die Umlaufrate des Reaktionsgemisches im Umlauf 3 betrug 300 m³/h. Bei einer Temperatur des Reaktionsgemisches von 40°C wurde innerhalb von 60 min bei konstanter Dosierrate insgesamt 6620 kg einer 50 %igen von Formaldehyd in Wasser über eine Mischdüse als Mischorgan 6 in den Kreislauf zugegeben. Die Temperierung des Reaktionsgemisches erfolgte durch einen Wärmeaustauscher 9. Nach der vollständigen Zugabe des Formaldehydes wurde das Reaktionsgemisch auf 90°C temperiert und anschließend in einen Vorratsbehälter mit einem Volumen von 70 m³ gefüllt. Von diesem Vorratsbehälter wurde das Reaktionsgemisch durch eine Temperiereinrichtung, mit der eine Temperatur des Reaktionsgemisches von 130°C eingestellt wurde, in einen Rohrreaktor überführt. Die Verweilzeit im Rohrreaktor betrug 150 min. Anschließend wurde das Gemisch bei einer Temperatur von 103°C mit 50 %iger wäßriger Natronlauge neutralisiert und die organische Phase von der wäßrigen Phase getrennt. Zur Entfernung von anorganischen Verunreinigungen bei einer Temperatur von 95°C mit Wasser gewaschen und die organische Phase von der wäßrigen Phase getrennt. Überschüssiges Anilin wurde aus der organischen Phase bei einer Temperatur von 180 bis 240°C und einem Druck von 1050 bis 5 mbar in einer dreistufigen Destillation entfernt.

Das somit erhaltene MDA wies einen N-Methyl MDA-Gehalt von 0,09 Gew.-% auf, der Gehalt an hydrolyisierbarem Chlor betrug 0,04 ppm. Das MDA wurde anschließend bei einer Temperatur von 80°C und einem Druck von 1,5 bar in einer Rührkesselkaskade mit einer Verweilzeit von 60 min mit Phosgen umgesetzt. Das molare Verhältnis von MDA zu Phosgen betrug 1:5,2. Die Phosgenierung wurde in Gegenwart von 15 Gew.-% Monochlorbenzol, bezogen auf das Gesamtgewicht des Reaktionsgemisches, durchgeführt. Nach der Phosgenierung wurden HCl und Phosgen bei einer Temperatur von 138°C und einem Druck von 1,2 bar entfernt, anschließend Lösungsmittel und gegebenenfalls chlorhaltige Verbindungen bei einer Temperatur von 180°C und einem Druck von 70 mbar abgetrennt. Das erhaltene Roh-MDI wurde bei einem Druck von 6 mbar und einer Temperatur von 217°C destillativ aufgetrennt in Polymer-MDI (PMDI) und Monomer-MDI (2,2'-MDI, 2,4'-MDI und 4,4'-MDI). Das gewonnene PMDI wies einen Gehalt an hydrolysierbarem Chlor von 400 ppm und eine Iodfarbzahl in einer Verdünnung von 1:5 in Monochlorbenzol von 10 auf.

## Patentansprüche

1. Verfahren zur Herstellung von Methylendi(phenylisocyanat) durch Phosgenierung von Methylendi(phenylamin), hergestellt durch Umsetzung von Anilin mit Formaldehyd in Gegenwart saurer Katalysatoren, wobei man in einem halbkontinuierlichen Verfahren Anilin und gegebenenfalls sauren Katalysator vorlegt, Formaldehyd und gegebenenfalls sauren Katalysator durch ein Mischorgan in einen Kreislauf, in dem Anilin, gegebenenfalls saurer Katalysator und gegebenenfalls bereits zugegebenes Formaldehyd kreisläufig bewegt werden, einspeist und nach Einspeisung von mindestens 50 % der gesamt einzuspeisenden Formaldehydmenge die Reaktionsmischung auf eine Temperatur von größer 75°C temperiert, **dadurch gekennzeichnet, dass** man die Phosgenierung bei Temperaturen von 70 bis 120°C und einem Druck von 0,8 bis 5 bar in einer oder mehreren Stufen durchführt.

2. Verfahren gemäß Anspruch 1, **dadurch gekennzeichnet, dass** die Phosgenierung durchgeführt wird durch eine zweistufige Umsetzung in Gegenwart mindestens eines inerten organischen Lösungsmittels, wobei die erste Stufe der Phosgenierung in einem statischen Mischer und die zweite Stufe der Phosgenierung in einem Verweilzeitapparat durchgeführt wird.

3. Verfahren gemäß Anspruch 1, **dadurch gekennzeichnet, dass** die Temperatur bei der ersten Stufe der Phosgenierung 90 bis 120°C beträgt.

## Claims

1. A process for preparing methylenedi(phenylisocyanate) by phosgenation of methylenedi(phenylamine), prepared by reacting aniline with formaldehyde in the presence of acid catalysts, which comprises, in a semicontinuous process, introducing aniline with or without acid catalyst, feeding formaldehyde with or without acid catalyst through a mixing element into a circuit in which aniline with or without acid catalyst and with or without previously added formaldehyde is circulated and, after feeding in at least 50% of the total amount of formaldehyde to be fed in, heating the reaction mixture to a temperature above 75°C wherein the phosgenation is carried out at temperatures of from 70 to 120°C and a pressure of from 0.8 to 5 bar in one or more steps.

2. A process as claimed in claim 1, wherein the phosgenation is carried out by a two-step reaction in the presence of at least one inert organic solvent, the first phosgenation step being carried out in a static mixer and the second phosgenation step being carried out in a dwell-time apparatus.

3. A process as claimed in claim 1, wherein the temperature in the first phosgenation step is from 90 to 120°C.

## Revendications

1. Procédé de préparation de méthylènedi(phénylisocyanate) par phosgénation de méthylènedi(phénylamine), préparé par réaction d'aniline avec du formaldéhyde en présence de catalyseurs acides, dans lequel de l'aniline et, éventuellement, un catalyseur acide sont mis en présence dans un procédé semi-continu, du formaldéhyde et, éventuellement, un catalyseur acide, sont alimentés par un organe mélangeur dans un circuit dans lequel l'aniline, éventuellement un catalyseur acide et, éventuellement, du formaldéhyde déjà ajouté sont mis en circulation et, après alimentation d'au moins 50% de la quantité totale de formaldéhyde à amener, le mélange réactif est tempéré à une température de plus de 75°C, **caractérisé en ce que** l'on procède à la phosgénation à des températures de 70 à 120°C et à une pression de 0,8 à 5 bars en une ou plusieurs étapes.

2. Procédé selon la revendication 1, **caractérisé en ce que** la phosgénation est effectuée par une réaction en deux étapes en présence d'au moins un solvant organique inerte, la première étape de la phosgénation étant effectuée dans un mélangeur statique et la deuxième étape de la phosgénation dans un appareil à temps de séjour.

3. Procédé selon la revendication 1, **caractérisé en ce que** la température dans la première étape de la phosgénation atteint 90 à 120°C.
